# EUROPEAN PATENT SPECIFICATION

(11) **EP 4 573 897 B1**
(45) Date of publication and mention of the grant of the patent: **09.12.2026**
(21) Application number: 23220139.2
(22) Date of filing: 22.12.2023
(51) Int. Cl.: A01K 67/36

(54) **FARMING INSTALLATION FOR GROWING INSECTS**
LANDWIRTSCHAFTLICHE ANLAGE ZUM ZÜCHTEN VON INSEKTEN
INSTALLATION AGRICOLE POUR LA CULTURE D'INSECTES

(43) Date of publication of application: 25.06.2025
(73) Proprietor: Nasekomo AD, Obshtina Stolichna, 1151 Selo Lozen (BG)
(72) Inventor: Bolard, Marc Louis Raymond, 1407 Sofia (BG); Vasilev, Kamen Nikolaev, 1000 Sofia (BG)
(74) Representative: Fidal Innovation

(56) References cited:
- EP-A1- 3 578 043
- EP-A1- 3 979 793

## Description

### FIELD OF THE INVENTION

The present invention relates to farming installation for growing insects, and particularly to a farming installation for growing insects comprising a buffering system for storing larvae.

### BACKGROUND OF THE INVENTION

Some insects efficiently convert organic secondary streams into protein, which can be used for animal feeding. One solution to recycle these secondary biomasses is to set up a biological treatment by replicating at a large scale the natural process of organic biomass cycle. Such process is called bioconversion.

A whole farming cycle is made of 4 steps:
- Step 1: Organic secondary streams are collected from agricultural or food industry producers for example, like breweries or ethanol plants.
- Step 2: Insects are reared in a highly bio-secure environment and fed with the organic secondary streams.
- Step 3: Insects are collected and separated from the remaining frass.
- Step 4: In a dry rendering process, insects are dried, and a protein meal and a fatty fraction are separated and further processed into animal feed ingredients. In a wet rendering process, the insects may first be cooked and grinded and then a separation of the solids, oils and water is performed to finally dry the solid fraction.
- Step 5: On the other hand, the insect frass is extracted to be used as organic fertilizer for agricultural plantation.

Thus, the organic fertilizer is useful for vegetable farming, and the protein-rich meal and the insect oil are used as components of animal feed.

Such process makes it possible to artificially reproduce the complete natural cycle of organic matter. Some pollution and waste of resources may then be avoided by replicating the whole natural cycle.

Among the advantages of such process, the environmental impact is highly reduced, in terms of water consumption, agricultural land usage, feed consumption.

The above step 2 can be divided into two phases: A first phase 2A comprises a maturation of insect eggs until the larvae hatch and grow for a few days, for example five days. A second aging phase 2B in a substrate lasts for 4 to 15 days, for example 7 to 8 days, and results in mature larvae and frass. The present invention especially concerns a process occurring between phases 2A and 2B.

More precisely, for large scale facilities, it is beneficial that all the larvae entering step 2B may be at the same stage of growth to optimise the growing process, to minimize the overall cost and to facilitate the logistics.

It is known from document EP3578043 to provide a storage tank for storing living larvae up to 4 days. When hatched, the larvae are provided in a tank filed with water at a temperature comprised between 7°C and 15°C to stop their metabolism. The content of the tank is agitated regularly to avoid the formation of clusters of larvae. However, this solution is not fully satisfying as it exists a risk for the larvae to drown in the water during their storage. Furthermore, it may exist a need to store the larvae for more than 4 days.

Document EP3979793 describes a similar process where larvae are stored in a tank filled with water at a temperature comprised between 8°C and 16°C for about 12 to 60 hours. The larvae can them by conveyed to a buffer container filled with water at the same temperature for about 10 to 40hours. This solution is not fully satisfying as it exists a risk for the larvae to drown in the water during their storage. Furthermore, it may exist a need to store the larvae for more than 60 hours.

The present invention aims to solve at least partially the above-identified technical problem.

### BRIEF SUMMARY OF THE INVENTION

To this aim, the invention relates to a farming installation for growing insects, the farming installation comprising:
- A first location where a first phase of growing the insects is configured to occur, the first phase corresponding to the hatching of larvae from eggs and the growth of said hatched larvae in a container filled with substrate for a few days in first environmental conditions to obtain young larvae,
- A second location where a second phase of growing the insects is configured to occur, said second phase being configured to occur after the first phase, the second phase comprising the growth of said young larvae for some days in second environmental conditions,
- A buffering system, the buffering system comprising:
   - A temperature-controlled chamber, the temperature-controlled chamber being configured to host the young larvae after the first phase and before the second phase, the temperature-controlled chamber comprising a temperature controlling system, said temperature controlling system being configured to regulate a temperature in the temperature-controlled chamber,
- Wherein the temperature controlling system is configured to periodically modify the temperature inside the temperature-controlled chamber between a lowest temperature and a highest temperature, and wherein the temperature-controlled chamber is configured to host the larvae for a predetermined buffering period, the predetermined buffering period lasting between 3 and 10 days, and preferably between 5 and 7 days. The lowest temperature is a temperature at which the larvae enter a hibernation state, and the highest temperature is a temperature at which the larvae get out of their hibernation state and partially wake up as to intensify their metabolism to maintain their vitality.
According to different aspects, it is possible to provide the one and / or the other of the characteristics below taken alone or in combination

In an embodiment, the lowest temperature is comprised between 10°C and 14°C, and is preferably equal to 12°C, and wherein the highest temperature is comprised between 16°C and 20°C and is preferably equal to 18°C.

In an embodiment, the temperature controlling system is configured to raise the temperature inside the temperature-controlled chamber to the highest temperature at least once a day.

In an embodiment, the buffering system further comprises transportation means for transporting the young larvae from the temperature-controlled chamber to the second location where the second phase takes place, said transportation means comprising autonomous vehicles.

In an embodiment, the buffering system further comprises an automated transportation line configured to transport containers to be filled with the young larvae obtained after the first phase, said containers being configured to be filled at a filing zone of said automated transportation line, said automated transportation line being further configured to transport the containers filled with young larvae from the filing zone to the temperature-controlled chamber.

In an embodiment, the buffering system further comprises a sieving device configured to receive the substrate and the young larvae once the first phase is over, the sieving device being configured to sieve the young larvae from the substrate in which the first phase occurred.

In an embodiment, the containers are configured to be filled with young larvae exiting the sieving device.

In an embodiment, the buffering system further comprises a bathing device configured to dispense a cold solution to the young larvae before the hosting of said young larvae in the temperature-controlled chamber, said cold solution being at a temperature comprised between 8°C and 14°C.

In an embodiment, the bathing device comprises a bathing conveyor, said bathing conveyor being configured to convey the young larvae obtained after the first phase to the filing zone, said bathing device further comprising a solution dispensing device configured to sprinkle the cold solution on the young larvae as they are being conveyed by the bathing conveyor.

In an embodiment, the bathing conveyor of the bathing device is further configured to convey the young larvae exiting the sieving device to the filing zone of the automated transportation line.

In an embodiment, the buffering system further comprises a drying zone configured to dry the larvae after they have been dispensed with the cold solution.

In an embodiment, during the last day of the first phase the larvae are configured to not receive any food.

In an embodiment, the farming installation is fully automated.

### BRIEF DESCRIPTION OF THE DRAWINGS

Embodiments of the invention will be described below with reference to the drawings, described briefly below:
[Fig. 1] represents a schematic view of a part of a buffering system of an insect farming installation,
[Fig. 2] represents a schematic view of an embodiment of a buffering system of an insect farming installation.
[Fig. 3] represents a schematic view of another embodiment of a buffering system of an insect farming installation.

In the drawings, identical references designate identical or similar objects.

### DETAILED DESCRIPTION OF THE INVENTION

In general, the system and the process according described herein are adapted for Dipteran insects at the neonate stage and the first days of larval development, such as the Black Soldier Fly (Hermetica Illucens), but other insects and/or other stages could be considered.

The life cycle of an insect comprises three or four stages: the egg stage, the larval stage, the pupae stage and the adult or imaginal stage (adult insect and imago being synonymous).

As stated above, the phase of rearing the insects is divided in two phases. A first phase 2A comprises hatching of insect eggs and initial growth for a few days, for example five days. The second phase 2B then occurs in a substrate and lasts for 4 to 15 days, for example 7 to 8 days, and results in mature larvae and frass.

In an embodiment, the first phase 2A and the second phase 2B occur in two different places since the condition of growth may not be the same between the two phases.

Indeed, the environmental conditions required for an optimal development of the insects aren't the same at these two phases.

Hence, the transferring of the larvae from the place where the first phase occurs to a different place where the second phase occur may allow controlling drastically the condition of growth, leading to a better performance of the facility, especially regarding the number and quality of insects obtained at the end of the overall growth cycle.

After the implementation of the first phase 2A, the young larvae are transferred to a space where they will develop until they reach their intended commercial growth stage (i.e. the second phase 2B).

Generally, during the first phase 2A, newly born larvae (neonates) are placed in a determined quantity in an initial container 101 full of feed where they start their initial growth. The first phase 2A generally lasts between 1 and 5 days. The larvae at the end of the first phase 2A are called "young larvae" herein after.

Once the first phase 2A is over, the young larvae are transferred from the initial container 101 to a buffering system 100 where they can be stocked until the beginning of the second phase 2B.

Generally, the buffering system 100 comprises at least the plurality of containers 101, a sieving device 102 and a temperature-controlled chamber 110.

The buffering system 100 may also optionally comprise a bathing conveyor 104 and a drying device 105.

In a preferred embodiment, the buffering system 100 may be fully automatic and autonomous.

Hence, the buffering system 100 may comprise a plurality of robots or automated manipulators (e.g. robot with arm, cartesian robot, gantry robot, or else) and automated transportation lines 106 allowing the transfer of the young larvae from a component to another, for example but in a non-limitative way from the containers 101 to the sieving device, from the sieving device to the temperature-controlled chamber 110 and others.

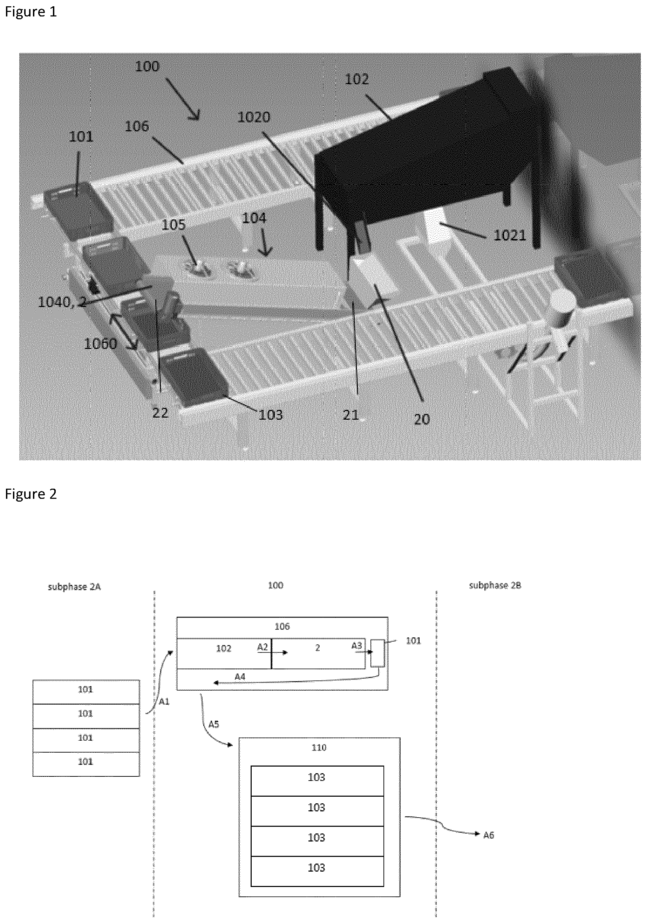

Figure 1 illustrates a part of the buffering system 100.

When the phase 2A is over, the container(s) 101 containing the young larvae are provided to a sieving device 102 configured to separate the young larvae from the substrate and/or frass in which they were growing during the first phase 2A.

Typically, such sieving device 102 may comprise a mesh configured to separate, under a movement such as a vibration, the larvae from the substrate and/or frass. The mesh may present holes being smaller than an average size of the larvae such that the larvae may not fall through the holes of the mesh. The young larvae and the substrate may be collected separately. In particular, the substrate and/or frass may be collected and directed toward another place for further treatment, while the young larvae continue the buffering process.

In order to provide automated and continuous process a robot (not shown) may take the containers 101 full of young larvae and may empty them into the inlet hopper of a sieving device 102. The robot then brings the empty containers 101 at the beginning of the automated transportation line 106, where they are subsequently filled with sieved young larvae, i.e. with young larvae that has been separated from the substrate and/or frass in the sieving device 102.

Hence, the containers 101 in which the first sub phase 2A occurred are used again to collect the sieved young larvae. When they are filled with sieved young larvae, the initial containers 101 are called buffering containers 103. To use the same containers during the first phase 2A and the buffering process is advantageous since the number of containers needed for the overall process is reduced. Optionally, the initial containers may be washed before being used to collect the sieved young larvae.

As visible on figure 1, the sieving device 102 comprises a larvae outlet 1020 through which the sieved young larvae are collected, and a frass outlet 1021 through which the frass and/or residues are collected for further treatment.

When the sieving has been done, the sieved young larvae fall from the sieving device 102, through the larvae outlet 1020 of the sieving device 102, down an insect transferring device 2, such as a moving belt conveyor or other.

More precisely, the insect transferring device 2 may present an entrance zone 21, where the young larvae fall from the sieving device 102, and an exit zone 22, where the young larvae fall in a buffering container 103.

In an embodiment, a hopper 20 may be provided between the larvae outlet 1020 of the sieving device 102 and the entrance zone 21 of the insect transferring device 2 to prevent the young larvae from falling on the ground instead of on the insect transferring device 2.

Due to the growing conditions provided in the plurality of initial containers 101, the young larvae contained in one initial container 101 grow approximately at the same speed. Advantageously, the initial containers 101 are each filled with young larvae of similar size and stage of development. Hence, the sieving device 102 may continuously be provided with young larvae, allowing the continuous filling of containers 101 with young larvae having approximately the same age.

In another embodiment, the containers 101 are not filled continuously but are filled from time to time, when the young larvae have grown for the required time and have reached similar stage of development, during step 2A.

As visible on figure 1, an automated transportation line 106 is provided to automatically transport the emptied initial containers 101 so that they are filled with sieved larvae, which end up as buffering containers 103.

The automated transportation line 106 may take the form of a conveyor belt, rollers or another suitable conveying system. The automated transportation line may be controlled by a controller (not shown) configured to command the activation of the automated transportation line 106. Upon receiving of the command, the automated transportation line 106 may start, then allowing the containers 101, 103 to move.

The plurality of initial 101 may be placed empty at the beginning of the automated transportation line 106. Then, when the automated transportation line starts, an empty initial container 101 is moved on a filing zone 1060 of the automated transportation line 106 where it is filled with sieved young larvae.

More precisely, the filing zone 1060 may be provided below the exit zone 22 of the insect transferring device 2.

An empty initial container 101 may be filled with between 100.000 larvae and 500.000 larvae. When filled with the sieved young larvae it becomes a buffering container 103.

To ensure this number of larvae per buffering container 103, the buffering system 100 may be provided with a counting device. In an embodiment, the counting device may be a scale. In an embodiment, the counting device is coupled to the controller. Hence, once the number of young larvae per buffering container is reached, the controller may send an activation command to the automated transportation line 106 which starts.

In an embodiment, the activation of the automated transportation line 106 is configured to allow a buffering container 103 full of young larvae to be moved a sufficient distance for an empty initial container 101 to end up at the filing zone 1060.

Optionally, the distance between two containers 101, 103 on the automated filing line 106 may be such that, once an empty initial container 101 is at the filing zone 1060, the buffering container 103 that preceded it arrives at the next station of the buffering system.

Figure 2 illustrates schematically the buffering system 100 comprising a temperature controlled chamber 110.

According to an embodiment, the buffering system 100 comprises a temperature-controlled chamber 110. More precisely, the temperature-controlled chamber 110 is configured to receive stacks of buffering containers 103 filled with young larvae, as described in relation with figure 1.

Buffering containers 103 are stacked by a robot and may be placed in the temperature-controlled chamber by an autonomous guided vehicle (AGV) or an autonomous mobile robot (AMR). The AGV/AMR may be configured to take a stack of buffering containers 103 coming out of the automated transportation line 106 of figure 1 and to put them in in the chamber 110, automatically.

The temperature-controlled chamber 110 comprises a temperature controlling system. The temperature controlling system may be used to control the temperature inside the temperature-controlled chamber hence to control the temperature of the larvae themselves.

The temperature controlling system of the temperature-controlled chamber 110 may comprise temperature and/or humidity controlling sensors. Such sensors may be coupled to a controller (not shown) to adapt the temperature in the chamber according to the measurements of the sensors, if needed.

In an embodiment, to maximize the thermic exchange between the interior of the temperature-controlled chamber 110 and the larvae, the containers 101, 103 may be made in a material having a high thermic capacity. For example, such material may be inox, aluminium, etc.

In another embodiment, the containers 101, 103 may be made in plastic.

As stated above, the growing process of larvae between first phase 2A and second phase 2B takes place in different places, possibly in different facilities, as the environment required to maximize their bioconversion is different in each of these phases.

In order to have a regular and acceptable rearing, it may be beneficial for a large number of larvae to begin second phase 2B at the same time, and even more beneficial that all the larvae beginning the second phase 2B to be at the same stage of growth. It will allow a better control of the growth of the larvae during phase 2B and an overall better performance of the insect growing installation.

To this aim, the temperature inside the temperature-controlled chamber 110 may be controlled by the temperature controlling system to oscillate periodically between a lowest temperature and a highest temperature.

In an embodiment, the lowest temperature may be comprised between 10°C and 14°C and is preferably equal to 12°C. At this temperature, the growth of the larvae is significantly slowed down or even completely stopped. This corresponds to a hibernation state of the larvae.

In an embodiment, the highest temperature may be comprised between 16°C and 20°C, and is preferably equal to 18°C. At this temperature, there is a partial restart of the metabolism of the young larvae that allows triggering the repair mechanism inside the body of the young larvae to mitigate the negative impact that the lowest temperature may cause to the bodies of the larvae during their stay in the temperature-controlled chamber.

In other word, the periodical oscillation of the temperature inside the temperature-controlled chamber allows to significantly slow down the growth of the young larvae, as they enter in a hibernation state while still keeping their vitality and maximizing survival rate after the process.

Advantageously, the temperature controlling system is configured to raise the temperature inside the temperature-controlled chamber to the highest temperature at least once a day. Advantageously, during the rest of the day, the temperature inside the temperature-controlled chamber is equal to the lowest temperature.

Advantageously, the temperature inside the temperature-controlled chamber is equal to the highest temperature for a period comprised between 1 hours and 4 hours a day, and preferably for a duration of 2 hours a day.

The duration of the stay of the young larvae inside the temperature-controlled chamber may last between 3 and 10 days, preferably between 5 and 7 days.

As stated above, during the stay of the young larvae in the temperature-controlled chamber, the temperature controlling system is configured to ensure the periodic change of the temperature in the temperature-controlled chamber.

As the temperature rises in the temperature-controlled chamber 110 until reaching the highest temperature, the young larvae will get out of their hibernation state and partially wake up. In this way they will temporarily intensify their metabolism, which will maintain their vitality. After the temporary increase of the temperature, the temperature is decreased again until reaching the lowest temperature to put the young larvae back in hibernation state.

Once there is a need to send a quantity of young larvae for rearing in phase 2B, the respective quantity of stacked buffering containers 103 can be extracted from the temperature-controlled chamber without disturbance of the stacks of young larvae which stay in the temperature-controlled chamber 110. As stated above, thanks to their staying in the temperature-controlled chamber 110, the second phase 2B will begin with a large number of young larvae being all in the same state of growth.

Advantageously, no food is given to the larvae while they are staying in the temperature-controlled chamber 110.

To enhance the buffering process of the larvae, some optional steps may further be implemented.

During the first phase 2A, a defined quantity of feed is given to the larvae so they can grow. The whole quantity of feed might be given at the beginning of phase 2A or there might be several feedings during this phase. A first optional step is to not give food to the larvae the last day of their growth during the first phase 2A. This leads to slowing down their growth. The young larvae are then delivered to the sieving device 102.

Another optional step is to bathe the young larvae in a cold solution before they enter the temperature-controlled chamber 110.

The temperature of the solution may be such as to allow cooling of the young larvae from their normal body temperature down to a hibernation temperature with least possible stress, for example between 8°C and 12°C.

The cold solution may be water. In another embodiment, the cold solution may be water plus citric acids. The citric acids may help to protect the exoskeleton of the larvae during the hibernation phase.

The step of bathing the larvae in the cold solution before they enter the temperature-controlled chamber 110 may be beneficial as the water (i.e. the cold solution) to water (i.e. the larvae are mostly composed by water molecules) thermic exchange is more efficient than only an air (i.e. the air of the temperature-controlled chamber) to water (i.e. the larvae's bodies) thermic exchange.

The step of bathing the larvae may be implemented by a bathing device 104 comprising a bathing conveyor 1040 and a cold solution dispensing device (not shown).

In an embodiment, the bathing conveyor 1040 may replace the insect transferring device 2. Hence, the bathing conveyor 1040 may receive young larvae from the sieving device 102 and may be configured to transport them to the filing zone 1060.

The cold solution dispensing device may comprise nozzles configured to sprinkle the cold solution onto the young larvae as they are transferred by said bathing conveyor. The bathing device 104 may further comprise a temperature controlling system (not shown) configured to regulate the temperature of the cold solution sprinkled onto the larvae such that the cold solution remains at the desired temperature. The bathing device 104 may then comprise sensors configured to measure the temperature of the cold solution. The sensors may be coupled to a controller, itself coupled to the temperature controlling system of the bathing device. If the temperature of the cold solution, measured by the sensor, is not the wanted temperature, the controller may send a command to the temperature-controlling system of the bathing container 104 to regulate the temperature of the cold solution.

The cold solution dispensing device may sprinkle the cold solution onto the larvae as they are transported by the bath conveyor 1040.

If the step of bathing the larvae is implemented, there is a need to dry them before they enter the temperature-controlled chamber 110 as they may otherwise die from drowning if they enter the hibernation state wet.

Hence, consecutively to the sprinkling of the larvae with the cold solution, the larvae may enter a drying section 105 configured to dry the larvae.

Typically, the drying of the young larvae may be done by blowing warm air on them.

In a preferred embodiment, all of the process/system described herein is fully automated. More precisely, robots may be used to transfer the larvae from initial containers 101 to the sieving device 102, to move the empty initial containers 101 to the automated transportation line 106 where they are automatically filled with sieved young larvae, to order the buffering containers 103 in stacks and to move the stacks of buffering containers 103 to and from the temperature-controlled chamber 110.

Automated vehicles or robots or automated conveyors or another automated warehousing/material-handling equipment may then be used to transfer the buffering containers 103 from the temperature-controlled chamber 110 to the location where the second phase 2B takes place or to a transport device that can deliver them to such a remote location.

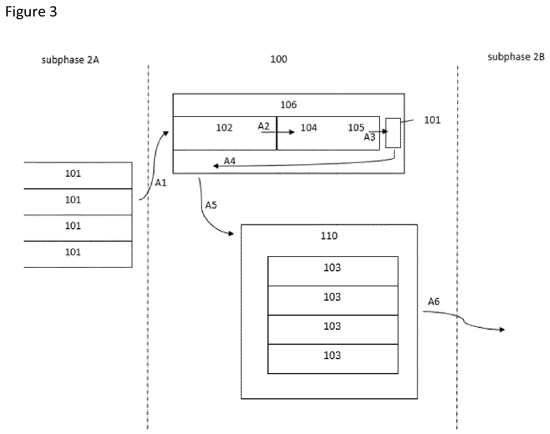

Figures 2 and 3 schematically show the process implemented by the buffering system. The arrows indicate the displacement of the young larvae in the different parts of the system.

As stated above, all the system is fully automated such that no human operator is involved in the implementation of the overall system.

On figure 2, a stack of initial containers 101 is shown. The initial containers 101 contain young larvae in substrate, obtained during the first phase 2A. Once the first phase 2A is finished, the young larvae are directed toward the buffering system 100 as represented by the arrow A1.

More precisely, a robot may take an initial container 101 full of young larvae and substrate and rotate it such that the content of the initial container 101 falls in the sieving device 102. The robot may then place the empty initial container 101 on the automated transportation line 106.

Once the sieving is performed, the sieved young larvae pass through the larvae sieving exit 1020 and fall on the insect transferring device 2, as represented by arrow A2.

The young larvae are then conveyed by the insect transferring device 2 to a filing zone 1060 of the automated transportation line 106. They advantageously fall from an exit of the transferring device 2 to an empty container 101 placed at the filling zone 1060, as represented by arrow A3.

The automated transportation line 106 advantageously transport empty container 101 disposed herein by the robot to the filling zone 1060 where the empty container 101 will be filed with sieved young larvae and becomes a buffering container 103. The automated transportation line 106 is further configured to transport the buffering containers 103 to the end of the transportation line (as represented by arrow A4).

Here, another robot, or the same robot, is configured to grab the buffering container 103 to put it in the temperature-controlled chamber 110, as represented by arrow A5.

Advantageously, the robot is configured to stack a plurality of buffering containers 103 in the temperature-controlled chamber 110.

Once required, another, or the same, robot may grasp a stack of buffering container to, either direct them in the location where the second phase 2B occurs, or to put them on an automated vehicle configured to move the stack of buffering containers 103 where the second phase occurs, as represented by arrow A6.

Figure 3 differs from figure 2 in that the optional step of bathing the young larvae occurs. Hence, instead of having an insect transferring device 102, the system 100 comprises a bathing device 104 comprising a bathing conveyor 1040 configured to direct the larvae from the exit of the sieving device 102 to the filing zone 1060. Advantageously, the bathing device comprises a cold solution dispensing device configured to sprinkle the cold solution on the larvae as they are moved by the bath conveyor. Advantageously, before entering in the filing zone, the larvae pass through a drying zone 105 configured to dry the larvae so they are not wet when placed in the buffering container 103.

While exemplary embodiment of the invention has been described with reference to the embodiments, it will be understood by those skilled in the art that various changes, omissions and/or additions may be made, and equivalents may be substituted for elements thereof without departing from the scope of the invention as defined in the appended claims. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. A farming installation for growing insects, the farming installation comprising:
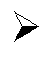 A first location where a first phase of growing the insects is configured to occur, the first phase corresponding to the hatching of larvae from eggs and the growth of said hatched larvae in a container (101) filled with substrate for a few days in first environmental conditions to obtain young larvae, 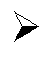
A second location where a second phase of growing the insects is configured to occur, said second phase being configured to occur after the first phase, the second phase comprising the growth of said young larvae for some days in second environmental conditions,
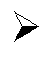 A buffering system (100), the buffering system (100) comprising:
∘ A temperature-controlled chamber (110), the temperature-controlled chamber (110) being configured to host the young larvae after the first phase and before the second phase, the temperature-controlled chamber (110) comprising a temperature controlling system, said temperature controlling system being configured to regulate a temperature in the temperature-controlled chamber (110),
**Characterized in that** the temperature controlling system is configured to periodically modify the temperature inside the temperature-controlled chamber (110) between a lowest temperature and a highest temperature, and wherein the temperature-controlled chamber (110) is configured to host the larvae for a predetermined buffering period, the predetermined buffering period lasting between 3 and 10 days, and preferably between 5 and 7 days, wherein the lowest temperature is a temperature at which the larvae enter a hibernation state, and wherein the highest temperature is a temperature at which the larvae get out of their hibernation state and partially wake up as to intensify their metabolism to maintain their vitality.

2. Farming installation according to claim 1, wherein the lowest temperature is comprised between 10°C and 14°C, and is preferably equal to 12°C, and wherein the highest temperature is comprised between 16°C and 20°C and is preferably equal to 18°C.

3. Farming installation according to claim 1 or 2, wherein the temperature controlling system is configured to raise the temperature inside the temperature-controlled chamber (110) to the highest temperature at least once a day.

4. Farming installation according to any of claims 1 to 3, wherein the buffering system (100) further comprises automated transportation means for transporting the young larvae from the temperature-controlled chamber (110) to the second location where the second phase takes place, for example said transportation means comprising autonomous vehicles.

5. Farming installation according to any of the preceding claims 1 to 4, wherein the buffering system (100) further comprises an automated transportation line (106) configured to transport other containers (101) to be filled with the young larvae obtained after the first phase, said containers (101) being configured to be filled at a filing zone (1060) of said automated transportation line (106), said automated transportation line (106) being further configured to transport the containers (103) filled with young larvae from the filing zone (1060) to the temperature-controlled chamber (110).

6. Farming installation according to any of claims 1 to 5, wherein the buffering system (100) further comprises a sieving device (102) configured to receive the substrate and the young larvae once the first phase is over, the sieving device (102) being configured to sieve the young larvae from the substrate in which the first phase occurred.

7. Farming installation according to claims 5 and 6, wherein the other containers are configured to be filled with young larvae exiting the sieving device.

8. Farming installation according to any of claims 1 to 7, wherein the buffering system (100) further comprises a bathing device (104) configured to dispense a cold solution to the young larvae before the hosting of said young larvae in the temperature-controlled chamber (110), said cold solution being at a temperature comprised between 8°C and 14°C.

9. Farming installation according to the preceding claim and to claim 5, wherein the bathing device (104) comprises a bathing conveyor (1040), said bathing conveyor (1040) being configured to convey the young larvae obtained after the first phase to the filing zone (1060), said bathing device (104) further comprising a solution dispensing device configured to sprinkle the cold solution on the young larvae as they are being conveyed by the bathing conveyor (1040).

10. Farming installation according to the preceding claim and to claim 7, wherein the bathing conveyor (1040) of the bathing device (104) is further configured to convey the young larvae exiting the sieving device (102) to the filing zone of the automated transportation line.

11. Farming installation according to any of claims 8 to 10, wherein the buffering system (100) further comprises a drying zone (105) configured to dry the larvae after they have been dispensed with the cold solution.

12. Farming installation according to any of the preceding claims, wherein the farming installation is configured such that during the last day of the first phase the larvae do not receive any food.

13. Farming installation according to any of the preceding claims, **characterized in that** the farming installation is fully automated by means of a plurality of robots or automated manipulators.

## Patentansprüche

1. Eine Insektenzuchtanlage zum Züchten von Insekten, wobei die Insektenzuchtanlage umfasst:
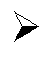 einen ersten Ort, an dem eine erste Phase der Aufzucht der Insekten stattfinden soll, wobei die erste Phase dem Schlüpfen von Larven aus Eiern und dem Wachstum der geschlüpften Larven in einem mit Substrat gefüllten Behälter (101) über einige Tage unter ersten Umgebungsbedingungen entspricht, um junge Larven zu erhalten,
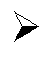 einen zweiten Ort, an dem eine zweite Phase der Aufzucht der Insekten stattfinden soll, wobei die zweite Phase nach der ersten Phase stattfinden soll und das Wachstum der jungen Larven über einige Tage unter zweiten Umgebungsbedingungen umfasst,
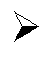 ein Puffersystem (100), wobei das Puffersystem (100) umfasst:
∘ eine temperaturgeregelte Kammer (110), wobei die temperaturgeregelte Kammer (110) dazu ausgelegt ist, die jungen Larven nach der ersten Phase und vor der zweiten Phase aufzunehmen, wobei die temperaturgeregelte Kammer (110) ein Temperaturregelsystem umfasst, wobei das Temperaturregelsystem dazu ausgelegt ist, eine Temperatur in der temperaturgeregelten Kammer (110) zu regeln,
**dadurch gekennzeichnet, dass** das Temperaturregelsystem dazu ausgelegt ist, die Temperatur innerhalb der temperaturgeregelten Kammer (110) periodisch zwischen einer niedrigsten Temperatur und einer Höchsttemperatur zu verändern, und wobei die temperaturgeregelte Kammer (110) dazu ausgelegt ist, die Larven während einer vorbestimmten Pufferdauer aufzunehmen, wobei die vorbestimmte Pufferdauer zwischen 3 und 10 Tagen und vorzugsweise zwischen 5 und 7 Tagen liegt, wobei die niedrigste Temperatur eine Temperatur ist, bei der die Larven in einen Überwinterungszustand eintreten, und wobei die Höchsttemperatur eine Temperatur ist, bei der die Larven aus ihrem Überwinterungszustand herauskommen und teilweise aufwachen, um ihren Stoffwechsel zu intensivieren und ihre Vitalität aufrechtzuerhalten.

2. Insektenzuchtanlage nach Anspruch 1, wobei die niedrigste Temperatur zwischen 10 °C und 14 °C liegt und vorzugsweise 12 °C beträgt, und wobei die Höchsttemperatur zwischen 16 °C und 20 °C liegt und vorzugsweise 18 °C beträgt.

3. Insektenzuchtanlage nach Anspruch 1 oder 2, wobei das Temperaturregelsystem dazu ausgelegt ist, die Temperatur innerhalb der temperaturgeregelten Kammer (110) mindestens einmal täglich auf die Höchsttemperatur anzuheben.

4. Insektenzuchtanlage nach einem der Ansprüche 1 bis 3, wobei das Puffersystem (100) ferner automatisierte Transportmittel zum Transportieren der jungen Larven von der temperaturgeregelten Kammer (110) zu dem zweiten Ort, an dem die zweite Phase stattfindet, umfasst, wobei die Transportmittel beispielsweise autonome Fahrzeuge umfassen.

5. Insektenzuchtanlage nach einem der vorhergehenden Ansprüche 1 bis 4, wobei das Puffersystem (100) ferner eine automatisierte Transportlinie (106) umfasst, die dazu ausgelegt ist, weitere Behälter (101) zu transportieren, die mit den nach der ersten Phase erhaltenen jungen Larven zu befüllen sind, wobei die Behälter (101) dazu ausgelegt sind, an einer Befüllzone (1060) der automatisierten Transportlinie (106) befüllt zu werden, wobei die automatisierte Transportlinie (106) ferner dazu ausgelegt ist, die mit jungen Larven gefüllten Behälter (103) von der Befüllzone (1060) zur temperaturgeregelten Kammer (110) zu transportieren.

6. Insektenzuchtanlage nach einem der Ansprüche 1 bis 5, wobei das Puffersystem (100) ferner eine Siebvorrichtung (102) umfasst, die dazu konfiguriert ist, das Substrat und die jungen Larven aufzunehmen, sobald die erste Phase beendet ist, wobei die Siebvorrichtung (102) dazu konfiguriert ist, die jungen Larven aus dem Substrat zu sieben, in dem die erste Phase stattgefunden hat.

7. Insektenzuchtanlage nach den Ansprüchen 5 und 6, wobei die weiteren Behälter dazu konfiguriert sind, mit jungen Larven befüllt zu werden, die aus der Siebvorrichtung austreten.

8. Insektenzuchtanlage nach einem der Ansprüche 1 bis 7, wobei das Puffersystem (100) ferner eine Badevorrichtung (104) umfasst, die dazu konfiguriert ist, den jungen Larven vor der Unterbringung der jungen Larven in der temperaturgeregelten Kammer (110) eine kalte Lösung zu verabreichen, wobei die kalte Lösung eine Temperatur zwischen 8 °C und 14 °C aufweist.

9. Insektenzuchtanlage nach dem vorhergehenden Anspruch und nach Anspruch 5, wobei die Badevorrichtung (104) einen Badeförderer (1040) umfasst, wobei der Badeförderer (1040) dazu konfiguriert ist, die nach der ersten Phase erhaltenen jungen Larven zur Befüllzone (1060) zu befördern, wobei die Badevorrichtung (104) ferner eine Lösungsabgabevorrichtung umfasst, die dazu konfiguriert ist, die kalte Lösung auf die jungen Larven zu sprühen, während diese durch den Badeförderer (1040) befördert werden.

10. Insektenzuchtanlage nach dem vorhergehenden Anspruch und nach Anspruch 7, wobei der Badeförderer (1040) der Badevorrichtung (104) ferner dazu konfiguriert ist, die aus der Siebvorrichtung (102) austretenden jungen Larven zur Befüllzone der automatisierten Transportlinie zu befördern.

11. Insektenzuchtanlage nach einem der Ansprüche 8 bis 10, wobei das Puffersystem (100) ferner eine Trockenzone (105) umfasst, die dazu konfiguriert ist, die Larven zu trocknen, nachdem ihnen die kalte Lösung verabreicht worden ist.

12. Insektenzuchtanlage nach einem der vorhergehenden Ansprüche, wobei Z r die Insektenzuchtanlage so konfiguriert ist, dass die Larven während des letzten Tages der ersten Phase keine Nahrung erhalten.

13. Insektenzuchtanlage nach einem der vorhergehenden Ansprüche, **dadurch gekennzeichnet, dass** die Insektenzuchtanlage mittels einer Vielzahl von Robotern oder automatisierten Manipulatoren vollständig automatisiert ist.

## Revendications

1. Installation d'élevage d'insectes destinée à l'élevage d'insectes, ladite installation d'élevage d'insectes comprenant :
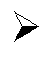 un premier emplacement où doit se dérouler une première phase d'élevage des insectes, ladite première phase correspondant à l'éclosion des larves à partir des œufs et à la croissance des larves écloses dans un récipient (101) rempli de substrat pendant plusieurs jours, dans des premières conditions environnementales, afin d'obtenir de jeunes larves,
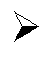 un deuxième emplacement destiné à accueillir une deuxième phase d'élevage des insectes, ladite deuxième phase devant avoir lieu après la première phase et comprenant la croissance des jeunes larves pendant plusieurs jours dans des deuxièmes conditions environnementales,
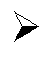 un système tampon (100), ledit système tampon (100) comprenant :
∘ une chambre à température contrôlée (110), ladite chambre à température contrôlée (110) étant conçue pour accueillir les jeunes larves après la première phase et avant la deuxième phase, ladite chambre à température contrôlée (110) comprenant un système de régulation de température, ledit système de régulation de température étant conçu pour réguler une température à l'intérieur de la chambre à température contrôlée (110),
**caractérisé en ce que** le système de régulation de température est conçu pour faire varier périodiquement la température à l'intérieur de la chambre à température contrôlée (110) entre une température minimale et une température maximale, et la chambre à température contrôlée (110) étant conçue pour accueillir les larves pendant une durée tampon prédéterminée, ladite durée tampon prédéterminée étant comprise entre 3 et 10 jours et de préférence entre 5 et 7 jours, la température minimale étant une température à laquelle les larves entrent en état d'hibernation, et la température maximale étant une température à laquelle les larves sortent de leur état d'hibernation et se réveillent partiellement afin d'intensifier leur métabolisme et de maintenir leur vitalité.

2. Installation d'élevage d'insectes selon la revendication 1, dans laquelle la température minimale est comprise entre 10 °C et 14 °C et est de préférence de 12 °C, et dans laquelle la température maximale est comprise entre 16 °C et 20 °C et est de préférence de 18 °C.

3. Installation d'élevage d'insectes selon la revendication 1 ou 2, dans laquelle le système de régulation de la température est conçu pour élever la température à l'intérieur de la chambre à température contrôlée (110) à la température maximale au moins une fois par jour.

4. Installation d'élevage d'insectes selon l'une des revendications 1 à 3, dans laquelle le système tampon (100) comprend en outre des moyens de transport automatisés pour transporter les jeunes larves de la chambre à température contrôlée (110) vers le deuxième emplacement où se déroule la deuxième phase, lesdits moyens de transport comprenant par exemple des véhicules autonomes.

5. Installation d'élevage d'insectes selon l'une des revendications 1 à 4 précédentes, dans laquelle le système tampon (100) comprend en outre une ligne de transport automatisée (106) conçue pour transporter d'autres récipients (101) destinés à être remplis avec les jeunes larves obtenues à l'issue de la première phase, les récipients (101) sont conçus pour être remplis au niveau d'une zone de remplissage (1060) de la ligne de transport automatisée (106), la ligne de transport automatisée (106) étant en outre conçue pour transporter les récipients (103) remplis de jeunes larves de la zone de remplissage (1060) vers la chambre à température contrôlée (110).

6. Installation d'élevage d'insectes selon l'une des revendications 1 à 5, dans laquelle le système tampon (100) comprend en outre un dispositif de tamisage (102) configuré pour recevoir le substrat et les jeunes larves dès que la première phase est terminée, le dispositif de tamisage (102) étant configuré pour tamiser les jeunes larves hors du substrat dans lequel la première phase a eu lieu.

7. Installation d'élevage d'insectes selon les revendications 5 et 6, dans laquelle les autres récipients sont configurés pour être remplis de jeunes larves sortant du dispositif de tamisage.

8. Installation d'élevage d'insectes selon l'une des revendications 1 à 7, dans laquelle le système tampon (100) comprend en outre un dispositif de bain (104) configuré pour administrer aux jeunes larves, avant leur placement dans la chambre à température contrôlée (110), la solution froide présentant une température comprise entre 8 °C et 14 °C.

9. Installation d'élevage d'insectes selon la revendication précédente et selon la revendication 5, dans laquelle le dispositif de bain (104) comprend un convoyeur de bain (1040), ledit convoyeur de bain (1040) étant configuré pour acheminer les jeunes larves obtenues après la première phase vers la zone de remplissage (1060), le dispositif de bain (104) comprenant en outre un dispositif de distribution de solution configuré pour pulvériser la solution froide sur les jeunes larves pendant qu'elles sont transportées par le convoyeur de bain (1040).

10. Installation d'élevage d'insectes selon la revendication précédente et selon la revendication 7, dans laquelle le convoyeur de bain (1040) du dispositif de bain (104) est en outre configuré pour acheminer les jeunes larves sortant du dispositif de tamisage (102) vers la zone de chargement de la ligne de transport automatisée.

11. Installation d'élevage d'insectes selon l'une des revendications 8 à 10, dans laquelle le système tampon (100) comprend en outre une zone de séchage (105) configurée pour sécher les larves après qu'on leur a administré la solution froide.

12. Installation d'élevage d'insectes selon l'une des revendications précédentes, dans laquelle l'installation d'élevage d'insectes est configurée de telle sorte que les larves ne reçoivent pas de nourriture au cours du dernier jour de la première phase.

13. Installation d'élevage d'insectes selon l'une des revendications précédentes, **caractérisée en ce que** l'installation d'élevage d'insectes est entièrement automatisée au moyen d'une pluralité de robots ou de manipulateurs automatisés.
